# FASCICULE DE BREVET EUROPEEN

(11) **EP 1 961 380 B1**
(45) Date de publication et mention de la délivrance du brevet: **23.06.2010**
(21) Numéro de dépôt: 08354002.1
(22) Date de dépôt: 16.01.2008
(51) Int. Cl.: A61B 5/107

(54) **Dispositif de mesure pour moignon fémoral**
Messvorrichtung für Oberschenkelstumpf
Measurement device for femoral stump

(30) Priorité: 20.02.2007 FR 0701205
(43) Date de publication de la demande: 27.08.2008
(73) Titulaire: Chabloz, Pierre, 38450 Saint Georges de Commiers (FR)
(72) Inventeur: Chabloz, Pierre, 38450 Saint Georges de Commiers (FR)
(74) Mandataire: Hecké, Gérard

(56) Documents cités:
- DE-U1- 9 200 810
- FR-A1- 2 403 776
- US-A- 4 974 331

## Description

### Domaine technique de l'invention

L'invention est relative à un dispositif de mesure pour moignon fémoral.

### État de la technique

L'élaboration d'une prothèse de jambe adaptée à un moignon fémoral comporte généralement une phase de prises de mesures sur le patient, et une phase de moulage pour fabriquer une emboîture dont le fût est adapté au moignon. La prise de mesures est classiquement réalisée le patient se tenant debout. Cette dernière série de mesures permet de tenir compte des déplacements de chairs molles par gravité.

Les mesures pratiquées avec le patient debout concernent la longueur du moignon, la distance séparant le sol et la branche ischio-pubienne du patient, et la mesure du périmètre du moignon dans des plans de coupe orthogonaux au fémur et espacés d'un intervalle prédéterminé.

De telles mesures sont délicates à réaliser et restent imprécises car elles sont pratiquées à l'aide d'un simple mètre ruban. Or, de telles imprécisions se répercutent directement sur la forme de l'emboîture de la prothèse réalisée par la suite, au détriment du confort du patient et de la fiabilité de la prothèse.

Le document FR-A1-2403776 divulgue un appareil de mesure pour relever les mensurations d'une jambe et pour déterminer la taille des diverses parties d'une jambe, par exemple la cuisse ou la cheville. L'appareil comporte une bande de longueur ajustable, une règle graduée, et des rubans munis d'une fermeture à boucles et à crochets de type Velcro ®.

Le document DE9200810U1 divulgue également un appareil pour mesurer le périmètre du fémur, qui comprend une ceinture de mesure qui est montée à coulissement le long d'une réglette verticale.

### Objet de l'invention

L'objet de l'invention consiste à réaliser un dispositif de mesure pour moignon fémoral qui soit de conception simple et peu onéreuse, et qui permette d'améliorer la précision des mesures réalisées à partir du patient.

Le dispositif de mesure selon l'invention est remarquable en ce qu'il comporte :
- un mât télescopique possédant des graduations correspondant à la distance entre ses première et deuxième extrémités,
- une réglette ayant une extrémité liée au mât télescopique par une articulation proche de la première extrémité dudit mât, la réglette possédant des graduations dont l'origine coïncide avec la première extrémité du mât,
- une ceinture en boucle fermée à périmètre réglable, montée à coulissement le long de la réglette et possédant des graduations correspondant à son périmètre.

Le mât télescopique d'un dispositif de mesure selon l'invention est destiné à être positionné entre le sol et la branche ischio-pubienne d'un patient se tenant debout sur lequel des mesures doivent être réalisées. Le mât télescopique sert de béquille et la lecture de ses graduations correspondant à la distance entre ses extrémités donne accès à la distance séparant le sol et la branche ischio-pubienne du patient. La réglette est disposée contre le moignon fémoral grâce à l'articulation et la ceinture est ajustée au périmètre du moignon dans des plans successifs perpendiculaires au fémur. Pour chaque mesure, laquelle est réalisée dans un plan donné, les graduations correspondant au périmètre de la ceinture donnent accès au périmètre du moignon tandis que les graduations de la réglette donnent accès très précisément à la distance séparant ledit plan et la branche ischio-pubienne.

Selon un mode de réalisation préférentiel, le mât télescopique comporte un guide muni d'un repère fixe et un coulisseau mobile dans le guide selon une direction longitudinale de coulissement, le coulisseau étant muni de graduations positionnées longitudinalement sur le coulisseau de sorte qu'une position alignée du repère fixe et de l'une desdites graduations correspond à une distance entre les première et deuxième extrémités du mât télescopique égale à la valeur de ladite graduation.

D'autres caractéristiques techniques peuvent être utilisées isolément ou en combinaison :
- l'articulation de la réglette comporte deux axes de pivotement perpendiculaires entre eux,
- la ceinture est montée à coulissement selon une première direction dans un élément de guidage monté à coulissement selon une deuxième direction le long de la réglette, les première et deuxième directions étant perpendiculaires entre elles,
- la première extrémité du mât télescopique comporte un appui monté à rotation libre,
- la ceinture est constituée par un mètre semi-rigide,
- une équerre est montée à coulissement le long de la réglette.

### Description sommaire des dessins

D'autres avantages et caractéristiques ressortiront plus clairement de la description qui va suivre d'un mode particulier de réalisation de l'invention donné à titre d'exemple non limitatif et représenté aux dessins annexés, dans lesquels :

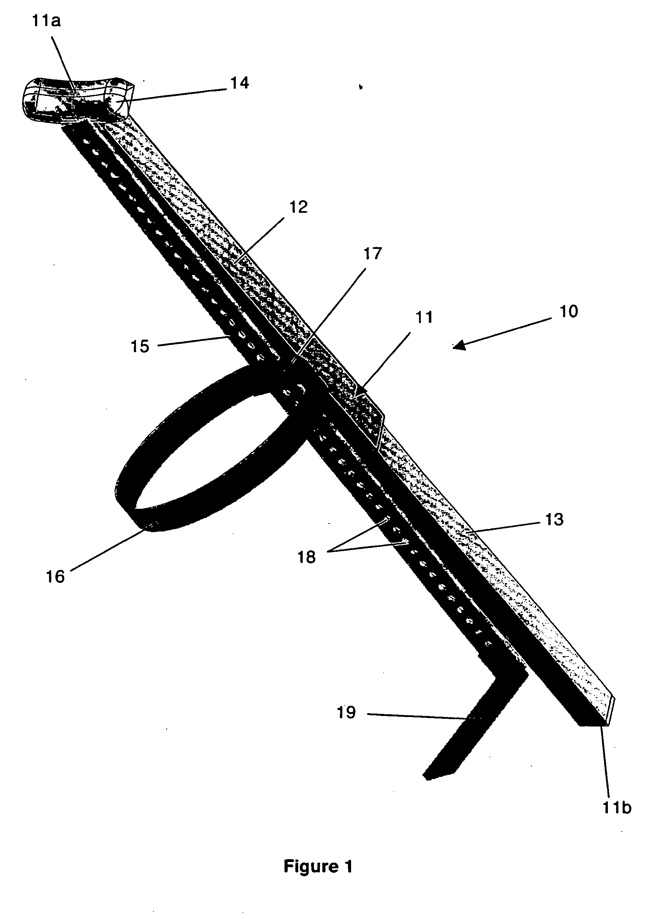

- la figure 1 représente, en perspective, un exemple de dispositif de mesure selon l'invention,
- la figure 2 est une vue de face du dispositif de la figure 1,
- la figure 3 est une vue de côté du dispositif des figures 1 et 2 après un pivotement de la réglette d'un angle ϕ1 autour d'un premier axe de pivotement,
- la figure 4 est une vue de face du dispositif de la figure 3,
- la figure 5 est une vue de face du dispositif des figures 1 et 2 après un pivotement de la réglette d'un angle ϕ2 autour d'un deuxième axe de pivotement.

### Description d'un mode préférentiel de l'invention

En référence aux figures, l'exemple de dispositif de mesure 10 pour moignon fémoral comporte un mât télescopique 11 se composant d'un guide 12 et d'un coulisseau 13 mobile dans le guide 12 selon une direction longitudinale de coulissement. Le mât télescopique 11 comporte des graduations correspondant à la distance entre ses première et deuxième extrémités, repérées 11 a, 11 b respectivement. Par exemple, de telles graduations sont réalisées à l'aide d'un repère fixe (non représenté) solidaire du guide 12 et de graduations solidaires du coulisseau 13 et réalisées par exemple grâce à une règle millimétrée. Les graduations du coulisseau 13 sont positionnées longitudinalement sur le coulisseau 13 de sorte qu'une position alignée du repère fixe et de l'une des graduations correspond physiquement à une distance entre les extrémités 11 a et 11 b du mât télescopique 11 qui est égale à la valeur de la graduation alignée avec le repère fixe. La première extrémité 11 a du mât télescopique 11 est constitué par l'une des extrémités du guide 12 et son extrémité 11 b est constitué par l'une des extrémités du coulisseau 13. Les autres extrémités du guide 12 et du coulisseau 13 servent à l'engagement du coulisseau 13 dans le guide 12. Un dispositif de blocage (non représenté) assure un blocage commandé de la translation du coulisseau 13 dans le guide 12.

La première extrémité 11a du mât télescopique 11 comporte un appui 14 cintré en forme d'arc de cercle dont la concavité est tournée du côté opposé au mât télescopique 11. L'appui 14 est monté à rotation libre sur le guide 12 selon un axe de rotation coïncidant avec la direction de coulissement du coulisseau 13 dans le guide 12. L'appui 14 possède une forme adaptée à l'anatomie du corps humain au niveau de la branche ischio-pubienne avec laquelle il est destiné à venir en contact comme expliqué plus loin, et comporte à cet effet un rembourrage pour le confort du patient. La première extrémité 11a correspond géométriquement au creux de l'appui 14. Autrement dit, la hauteur du mât télescopique 11, indiquée par les graduations décrites ci-dessus, est comptée entre sa deuxième extrémité 11 b et le creux de l'appui 14. Une housse amovible à usage unique recouvre, pour des raisons d'hygiène, l'extrémité 11a.

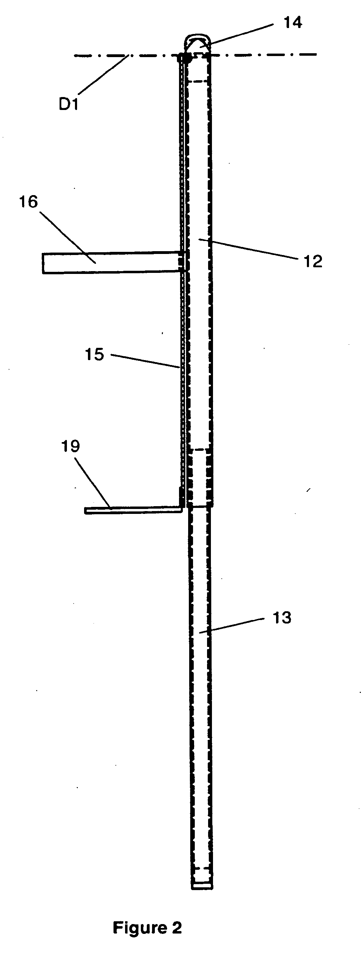

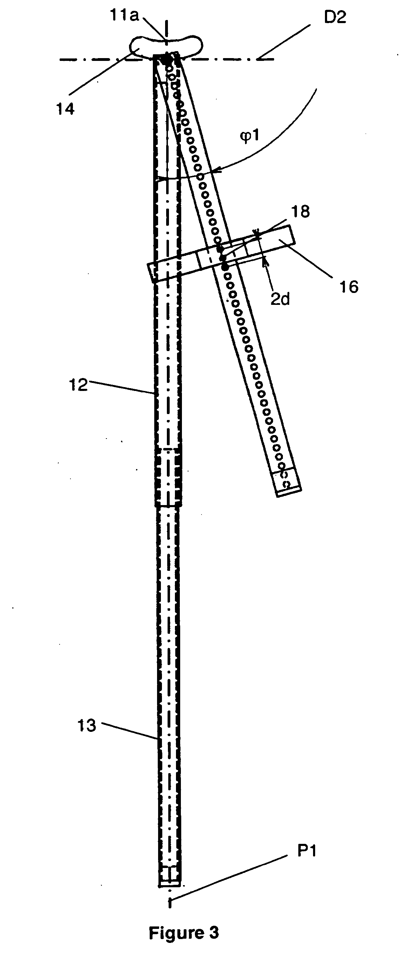

Le dispositif de mesure 10 comporte également une réglette 15 ayant une extrémité liée au guide 12 du mât télescopique 11 par une articulation proche de la première extrémité 11a. Cette articulation comporte deux axes de pivotement perpendiculaires entre eux. Ainsi, la réglette 15 est articulée à son extrémité par rapport au mât télescopique 11 autour d'un premier axe de pivotement D1 (figure 2) qui est perpendiculaire à la direction de coulissement du coulisseau 13 dans le guide 12. Dans un mouvement indépendant, la réglette 15 est aussi articulée à son extrémité par rapport au mât télescopique 11 autour d'un deuxième axe de pivotement D2 (figure 3) qui est simultanément perpendiculaire à D1 et à la direction de coulissement du coulisseau 13 dans le guide 12. Avec une telle articulation, la réglette 15 est montée, par rapport au mât télescopique 11, selon une liaison sphérique à doigt interdisant, à chaque instant, la rotation sur elle-même de la réglette 15. Le centre de cette liaison sphérique est défini par le point de concours des axes de pivotement D1 et D2. Une telle articulation peut, par exemple, être réalisée à l'aide d'un croisillon dont une première branche est montée à pivotement par rapport au guide 12 du mât télescopique 11, et l'autre branche par rapport à la réglette 15. Néanmoins toute réalisation équivalente pour parvenir au même résultat peut être envisagée.

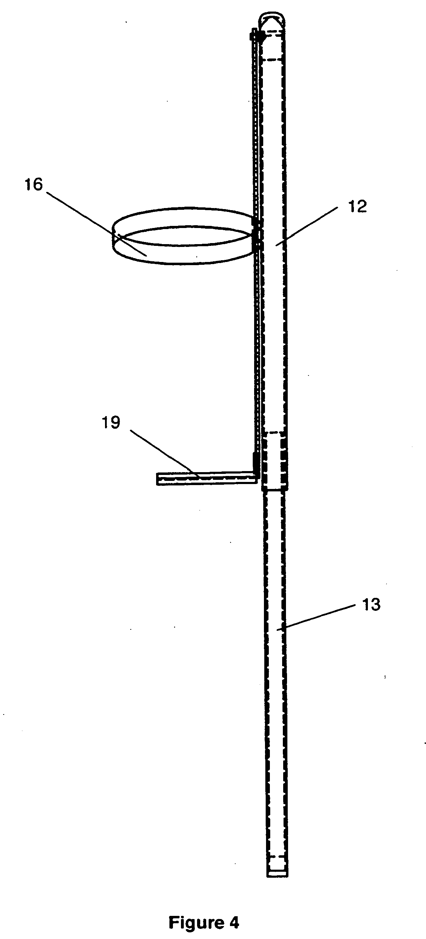

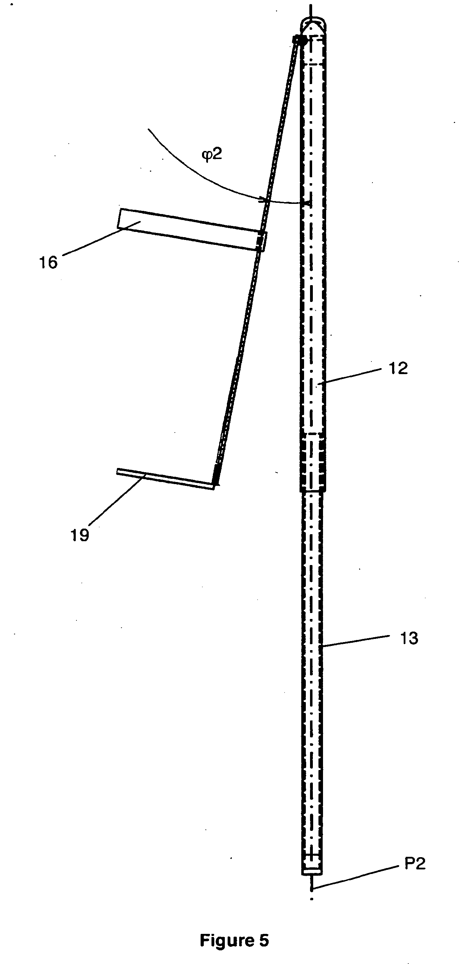

Les figures 3 à 5 illustrent le dispositif de mesure 10 pour différentes configurations de pivotement de la réglette 15. Sur les figures 3 et 4, la réglette 15 est représentée après un pivotement d'un angle ϕ1 autour du premier axe de pivotement D1. L'angle ϕ1 est mesuré à partir d'un premier plan P1 (figure 3) défini comme le plan contenant le premier axe de pivotement D1 et la direction de coulissement du coulisseau 13 dans le guide 12. Sur la figure 5, la réglette 15 est représentée après un pivotement d'un angle ϕ2 autour du deuxième axe de pivotement D2. L'angle ϕ2 est mesuré à partir d'un deuxième plan P2 (figure 5) défini comme le plan contenant le deuxième axe de pivotement D2 et la direction de coulissement du coulisseau 13 dans le guide 12. Par conséquent, le deuxième plan P2 constitue le plan dans lequel se produit le pivotement de la réglette 15 autour du premier axe de pivotement D1. De manière réciproque, le premier plan P1 constitue le plan dans lequel se produit le pivotement de la réglette 15 autour du deuxième axe de pivotement D2. Les plans P1 et P2 sont orthogonaux entre eux.

Conformément à l'invention, la réglette 15 possède des graduations dont l'origine coïncide avec la première extrémité 11a du mât télescopique 11. Pour y parvenir, la valeur de la première graduation de la réglette 15 (à partir de l'articulation de la réglette 15 sur le mât télescopique 11) correspond à la distance séparant ladite première graduation et la première extrémité 11a lorsque le mât 11 et la réglette 15 sont parallèles. Une telle réalisation permet d'inclure, dans la valeur des graduations, la distance séparant la première extrémité 11 a du mât 11 et l'articulation de la réglette 15 sur le mât 11. Les graduations peuvent être réalisées à l'aide d'une règle millimétrée dont le positionnement le long de la réglette 15 est fixe et choisi pour respecter les critères ci-dessus à propos de ses graduations. La réglette 15 peut être semi-rigide pour suivre la forme du moignon si celui-ci n'est pas rectiligne.

Le dispositif de mesure comporte aussi une ceinture 16 en boucle fermée à périmètre réglable. La ceinture 16 est montée à coulissement selon une première direction dans un élément de guidage 17 monté à coulissement selon une deuxième direction le long de la réglette 15. La première direction est perpendiculaire à la direction principale de la réglette 16 tandis que la deuxième direction correspond à la direction principale de la réglette 16. Les première et deuxième directions sont perpendiculaires entre elles. Une telle structure garantit que la ceinture 16 s'étend dans un plan perpendiculaire à la direction principale de la réglette 15 et que la ceinture 16 peut se translater le long de la réglette 15 par coulissement de l'élément de guidage 17. Une série de trous d'indexation 18 est pratiquée le long de la réglette 16 à intervalles réguliers repéré d (la figure 3 montre l'intervalle entre deux trous 18 non successifs égal à 2 fois la distance d) suivant sa direction principale. Les trous d'indexations 18 sont destinés à coopérer avec l'élément de guidage 17 par tout moyen connu. Lorsqu'ils coopèrent, la distance séparant la première extrémité 11 a du mât télescopique 11 et l'élément de guidage 17 supportant la ceinture 16 est fixe et correspond à une distance prédéterminée.

La ceinture 16 possède des graduations correspondant à son périmètre. Une telle ceinture 16 peut être constituée par un mètre semi-rigide, en métal ou en plastique par exemple. Dans une première variante, un premier brin de la ceinture 16 est solidaire de l'élément de guidage 17 et l'autre brin, le deuxième, est coulissant dans l'élément de guidage 17 selon la deuxième direction. Dans ce cas, l'élément de guidage 17 permet la lecture sur le deuxième brin de la graduation qui coïncide avec l'origine des graduations sur le premier brin. La valeur lue est égale au périmètre de la ceinture 16. Dans une deuxième variante, le premier brin est solidaire d'une boucle (non représentée) et le deuxième brin est monté coulissant dans la boucle. À proximité de son milieu, la ceinture 16 est montée à coulissement dans l'élément de guidage 17. Dans cette variante, la boucle permet la lecture sur le deuxième brin de la graduation qui coïncide avec l'origine des graduations sur le premier brin. Quelle que soit la variante, la lecture doit pouvoir se faire sur le côté diamétralement opposé à la réglette 15 pour des raisons de facilité de lecture.

Pour finir, le dispositif de mesure 10 comporte une équerre 19 qui est montée à coulissement le long de la réglette 15. Les trous d'indexations 18 ne sont pas destinés à coopérer avec l'équerre 19. La distance séparant la première extrémité 11 a du mât télescopique 11 et l'équerre 19 est variable et le déplacement de l'équerre 19 est commandé manuellement. L'équerre 19 est perpendiculaire simultanément à la première direction de coulissement de la ceinture 16 dans l'élément de guidage 17 et à la deuxième direction de coulissement de l'élément de guidage 17 le long de la réglette 15.

### L'utilisation du dispositif de mesure 10 est la suivante :

Le mât télescopique 11 est placé verticalement sous le patient qui se tient debout, entre le sol et la branche ischio-pubienne du patient du côté de l'amputation. Le mât 11 est orienté de telle manière que le deuxième plan P2 coïncide avec le plan sagittal du patient. Le plan sagittal est défini comme le plan vertical orienté dans le sens antéro-postérieur sur la ligne médiane du corps du patient. Le premier plan P1 coïncide alors avec le plan médio-latéral frontal du patient.

Un ajustement de la longueur du mât 11 est souvent nécessaire et se pratique à l'aide du dispositif de blocage prévu pour la commande du mouvement du coulisseau 13. Le dispositif de blocage est d'abord déverrouillé pour pouvoir translater le coulisseau 13 dans le guide 12 jusqu'à la mise en contact de l'appui 14 contre la branche ischio-pubienne du patient. Puis le dispositif de blocage est verrouillé pour interdire la translation du coulisseau 13. Par la suite, le patient peut reposer sur l'appui 14 de manière que le mât télescopique 11 constitue une béquille. La lecture de la valeur de la graduation du coulisseau 13 alignée avec le repère fixe du guide 12 donne accès à la distance séparant les extrémités 11 a, 11 b du mât 11, et donc à la distance séparant le sol et la branche ischio-pubienne du patient.

Puis la réglette 15 est orientée sensiblement parallèlement au moignon fémoral à mesurer sur le patient, grâce à l'articulation produisant une liaison sphérique à doigt. La réglette 15 vient en appui contre la face interne du moignon. La ceinture 16 est disposée autour du moignon à proximité de l'aine du patient, puis ajustée au périmètre du moignon. L'équerre 19 est ensuite coulissée le long de la réglette 15 jusqu'à venir en contact avec l'extrémité du moignon. La lecture de la graduation de la réglette 15 qui coïncide avec l'équerre 19 donne accès à la distance séparant l'équerre 19 et la première extrémité 11 a du mât télescopique 11, et donc à la longueur du moignon du patient, comptée à partir de sa branche ischio-pubienne.

Ensuite, la mesure du périmètre du moignon dans différents plans de coupe orthogonaux au fémur est réalisée. Lors de chaque mesure, la ceinture 16 est ajustée au moignon et la lecture de la graduation de la ceinture 16 correspondant à son périmètre donne accès au périmètre du moignon. La lecture de la graduation de la réglette 15 alignée avec l'élément de guidage 17 donne accès à la distance séparant la ceinture 16 et la première extrémité 11a du mât télescopique 11, et donc à la distance entre la ceinture 16 et la branche ischio-pubienne du patient. Grâce aux trous d'indexation 18, cette dernière distance appartient à un ensemble de valeurs prédéterminées et n'est donc pas aléatoire. Au contraire, elle est parfaitement quantifiable et de manière précise. Puis l'élément de guidage 17 est dégagé du trou d'indexation 18 avec lequel il coopère, puis est translaté le long de la réglette 16 dans la direction opposée à la liaison avec le mât télescopique 11 jusqu'à coopérer avec le trou d'indexation 18 suivant pour réaliser la mesure suivante. Les mesures du périmètre du moignon se font avec une incrémentation régulière de la distance séparant la ceinture 16 et la branche ischio-pubienne, selon un pas égal à l'intervalle entre deux trous d'indexation 18. Il va de soi que les mesures du périmètre du moignon peuvent être réalisées pour des positions de la ceinture 16 qui sont séparées par des intervalles égaux à un multiple de la distance entre deux trous d'indexation 18. Cette succession d'étapes permet facilement de connaître, au final, la valeur du périmètre du moignon dans un plan perpendiculaire au fémur en fonction de la distance très précise séparant ce plan et la branche ischio-pubienne du patient.

La précision des mesures réalisées à l'aide du dispositif de mesure 10 selon l'invention se répercute directement sur la qualité de l'emboîture de la prothèse réalisée à partir de ces mesures, au profit du confort du patient et de la fiabilité de la prothèse.

## Revendications

1. Dispositif de mesure (10) pour moignon fémoral, comportant les suivantes caractéristiques techniques:
- un mât télescopique (11) possédant des graduations correspondant à la distance entre ses première et deuxième extrémités (11a, 11b),
- une réglette (15) ayant une extrémité liée au mât télescopique (11) par une articulation proche de la première extrémité (11a) dudit mât (11), la réglette (15) possédant des graduations dont l'origine coïncide avec la première extrémité (11a) du mât (11),
- une ceinture (16) en boucle fermée à périmètre réglable, montée à coulissement le long de la réglette (15) et possédant des graduations correspondant à son périmètre.

2. Dispositif selon la revendication 1, **caractérisé en ce que** l'articulation de la réglette (15) comporte deux axes de pivotement (D1, D2) perpendiculaires entre eux.

3. Dispositif selon l'une des revendications 1 et 2, **caractérisé en ce que** la ceinture (16) est montée à coulissement selon une première direction dans un élément de guidage (17) monté à coulissement selon une deuxième direction le long de la réglette (15), les première et deuxième directions étant perpendiculaires entre elles.

4. Dispositif selon l'une des revendications 1 à 3, **caractérisé en ce que** la première extrémité (11a) du mât télescopique (11) comporte un appui (14) monté à rotation libre.

5. Dispositif selon l'une des revendications 1 à 4, **caractérisé en ce que** la ceinture (16) est constituée par un mètre semi-rigide.

6. Dispositif selon l'une des revendications 1 à 5, **caractérisé en ce que** le mât télescopique (11) comporte un guide (12) muni d'un repère fixe et un coulisseau (13) mobile dans le guide (12) selon une direction longitudinale de coulissement, le coulisseau (13) étant muni de graduations positionnées longitudinalement sur le coulisseau (13) de sorte qu'une position alignée du repère fixe et de l'une desdites graduations correspond à une distance entre les première et deuxième extrémités (11a, 11b) du mât télescopique (11) égale à la valeur de ladite graduation.

7. Dispositif selon l'une des revendications 1 à 6, **caractérisé en ce qu'**il comporte une équerre (19) montée à coulissement le long de la réglette (15).

## Claims

1. Measuring device (10) for a femoral stump comprising the following technical characteristics:
- a telescopic post (11) having graduations corresponding to the distance between its first and second ends (11a, 11b),
- a rule (15) having one end joined to the telescopic post (11) by a hinge-joint close to the first end (11a) of said post (11), the rule (15) having graduations the origin whereof coincides with the first end (11a) of the post (11),
- a belt (16) in the form of a closed loop with an adjustable perimeter fitted sliding along the rule (15) and having graduations corresponding to its perimeter.

2. Device according to claim 1, **characterized in that** the hinge-joint of the rule (15) comprises two swivel axes (D1, D2) perpendicular to one another.

3. Device according to one of claims 1 and 2, **characterized in that** the belt (16) is fitted sliding in a first direction in a guide element (17) mounted sliding in a second direction along the rule (15), the first and second directions being perpendicular to one another.

4. Device according to one of claims 1 to 3, **characterized in that** the first end (11a) of the telescopic post (11) comprises a bearing support (14) mounted rotating freely.

5. Device according to one of claims 1 to 4, **characterized in that** the belt (16) is formed by a semi-rigid tape strip.

6. Device according to one of claims 1 to 5, **characterized in that** the telescopic post (11) comprises a guide (12) equipped with a fixed mark and a slide (13) movable in the guide (12) in a longitudinal direction of sliding, the slide (13) being provided with graduations positioned longitudinally on the slide (13) so that an aligned position of the fixed mark and of one of said graduations corresponds to a distance between the first and second ends (11a, 11b) of the telescopic post (11) equal to the value of said graduation.

7. Device according to one of claims 1 to 6, **characterized in that** it comprises an angle bracket (19) mounted sliding along the rule (15).

## Patentansprüche

1. Messvorrichtung (10) für den Oberschenkelstumpf, die folgende technische Merkmale aufweist:
- einen Teleskopmast (11) mit Messeinteilungen, die dem Abstand zwischen seinem ersten und zweiten Ende (11a, 11b) entsprechen,
- eine Leiste (15), deren eines Ende mit dem Teleskopmast (11) über ein Gelenk verbunden ist, das sich nahe dem ersten Ende (11a) des genannten Masts (11) befindet, wobei die Leiste (15) Messeinteilungen aufweist, deren Anfang mit dem ersten Ende (11a) des Mast (11) zusammenfällt,
- einen geschlossenen Gurt (16) mit einstellbarem Umfang, der gleitend entlang der Leiste (15) montiert ist und Messeinteilungen umfasst, die seinem Umfang entsprechen.

2. Vorrichtung nach Anspruch 1, **dadurch gekennzeichnet, dass** das Gelenk der Leiste (15) zwei Schwenkachsen (D1, D2) umfasst, die lotrecht zueinander sind.

3. Vorrichtung nach einem der Ansprüche 1 und 2, **dadurch gekennzeichnet, dass** der Gurt (16) gleitend in einer ersten Richtung in einem Führungselement (17) montiert ist, das gleitend in einer zweiten Richtung entlang der Leiste (15) montiert ist, wobei die erste und die zweite Richtung lotrecht zueinander sind.

4. Vorrichtung nach einem der Ansprüche 1 bis 3, **dadurch gekennzeichnet, dass** das erste Ende (11a) des Teleskopmasts (11) eine Auflage (14) umfasst, die frei drehbar montiert ist.

5. Vorrichtung nach einem der Ansprüche 1 bis 4, **dadurch gekennzeichnet, dass** der Gurt (16) aus einem halbstarren Maßstab besteht.

6. Vorrichtung nach einem der Ansprüche 1 bis 5, **dadurch gekennzeichnet, dass** der Teleskopmast (11) eine Führung (12) umfasst, die mit einer festen Markierung und einem in der Führung (12) entlang einer Gleit-Längsrichtung beweglichen Schieber (13) versehen ist, wobei der Schieber (13) Messeinteilungen umfasst, die entlang dem Schieber (13) der Länge nach positioniert sind, sodass eine aufeinander ausgerichtete Position der festen Markierung und einer der genannten Messeinteilungen einem Abstand zwischen dem ersten und zweiten Ende (11a, 11b) des Teleskopmasts (11) entspricht, der gleich dem Wert der genannten Messeinteilung ist.

7. Vorrichtung nach einem der Ansprüche 1 bis 6, **dadurch gekennzeichnet, dass** sie einen Winkel (19) umfasst, der gleitend entlang der Leiste (15) montiert ist.
